Europäisches Patentamt

European Patent Office

Office européen des brevets

(19)

(11) Numéro de publication: **0 037 522**
**B1**

(12) **FASCICULE DE BREVET EUROPEEN**

(45) Date de publication du fascicule du brevet: **19.10.83**

(21) Numéro de dépôt: **81102264.9**

(22) Date de dépôt: **26.03.81**

(51) Int. Cl.³: **C 07 G 7/00,**
**A 61 K 39/108**

(54) Dérivés de l'entérotoxine thermostable de Escherichia Coli et leur préparation.

(30) Priorité: **04.04.80 US 137326**

(43) Date de publication de la demande:
**14.10.81 Bulletin 81/41**

(45) Mention de la délivrance du brevet:
**19.10.83 Bulletin 83/42**

(84) Etats contractants désignés:
**AT BE CH DE FR GB IT LI LU NL SE**

(56) Documents cités:
**DE - A - 2 137 630**
**FR - A - 2 270 891**

**BIOLOGICAL ABSTRACTS, vol. 70, no. 8, aoÛt
1980 PHILADELPHIA (US) & Acta Vet Acad.
Hung 28(i): 1—12 1980 I. PESTI et al.:
"Escherichia coli vaccine for prevention of
neonatal enteric colibacillosis in pigs", page
5365, abrégé no. 51054**

(73) Titulaire: **Smith Kline - RIT Société anonyme dite:
rue du Tilleul, 13
B-1320 Genval (Rixensart) (BE)**

(72) Inventeur: **van Wijnendaele, Frans
Teckerstraat, 12
B-3052 Ottenburg (BE)**

(74) Mandataire: **Tasset, Gérard
Smith Kline - RIT rue de l'Institut, 89
B-1330 Rixensart (BE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

# 0 037 522

Dérivés de l'entérotoxine thermostable de Escherichia coli et leur préparation

La présente invention concerne des nouveaux dérivés de l'entérotoxine thermostable de *E. coli,* un procédé pour leur préparation et des compositions prophylactiques antidiarrhéiques contenant lesdits dérivés de l'entérotoxine thermostable de *E. coli* et destinées à l'administration à l'homme et aux animaux.

Il est connu que les souches de Escherichia coli peuvent être responsables de la diarrhée aussi bien chez l'homme que chez les animaux et différentes études (p. ex. Gyles and Barnum J. Infect. Dis *120,* 419—426, 1969), ont démontré que l'entéropathogénicité de la souche de *E. coli* est liée à la production d'entérotoxines.

Deux entérotoxines ont été décrites: une entérotoxine thermolabile (LT)et une entérotoxine thermostable (ST) et Smith and Gyles (J. Med. Microbiol. *3,* 387—401, 1970) ont classifié un certain nombre de souches entéropathogènes de E. *coli* de différentes origines animales en tant que productrices d'entérotoxines LT et ST ou uniquement d'entérotoxine ST. Des souches de E. coli d'origine humaine et productrices d'entérotoxine ST ont également été décrites ou rapportées par différents auteurs. (p. ex. R. A. GIANNELLA, Infect. Immun. *14,* 95—99, 1976). L'entérotoxine thermolabile est sous le contrôle génétique d'un plasmide transmissible; elle paraît être constituée d'un groupe de peptides liés entre eux, d'un poids moléculaire d'environ 24.000. L'entérotoxine LT est adsorbée aux gangliosides à la bordure en brosse des cellules épithéliales de l'intestin grêle où, par l'intermédiaire d'un processus enzymatique, elle provoque une hypersécrétion d'eau et de de chlorures dans le tube intestinal et empêche la réabsorption de sodium, de sorte que le tube intestinal se dilate au passage du liquide, entraînant ainsi une hypermotilité et une diarrhée. L'entérotoxine thermolabile (LT) est antigénique et stimule des anticorps neutralisants dans le sérum de l'homme ou de l'animal préalablement infecté par une souche de *E. coli* productrice de toxine. L'entérotoxine thermostable (ST) est sous le contrôle génétique d'un groupe hétérogène de plasmides; elle provoque également la diarrhée, peut-être en stimulant la guanidine cyclase (J. M. Hughes et al., Nature *271,* 755—6, 1978). Comme il avait été démontré que l'antisérum préparé contre des organismes producteurs de LT et de ST neutralise la LT mais pas la ST et que les antisérums préparés de la même façon contre la ST n'ont pas d'effet neutralisant sur la ST ou la LT, Smith et Gyles (ref. citée) ont conclu que la ST n'est pas antigénique. Dans un article intitulé "Purification and chemical characterization of the heat stable enterotoxin produced by porcine strains of enterotoxigenic *Escherichia coli* (Infect. Immun. *19,* 1021—1030, 1978) J. F. Alderete et D. C. Robertson ont signalé que la ST purifiée "présentait un poids moléculaire de 4.400, lorsque déterminé à la fois par électrophorèse sur gel de dodécylsulfate de sodium et par filtration sur gel" et "un poids moléculaire de 5.100, représentant 47 résidus lorsque calculé à partir des données analytiques d'acides aminés" et ils ont également indiqué que "l'antisérum obtenu à partir de lapins immunisés avec de la ST ou de la ST couplée à de l'albumine de sérum bovin neutralisait l'action de l'entérotoxine chez les souriceaux à la mammelle; toutefois, des tests d'hémagglutination passive et du taux d'hémolyse semblaient indiquer que la ST est un médiocre antigène". La détermination du poids moléculaire de la ST effectuée par d'autres auteurs a conduit à des valeurs différentes et inférieures (p. ex. 1000—1200, 1500—2000 et 2500 daltons, comme indiqué par E.V. Lee at ai., R. Lallier et al. et G.L. Madsen et al. dans American Society of Microbiology meeting, respectivement 1973, résumé M 168, 1979, résumé B 34 et 1979, résumé P29), ces divergences pouvant peut-être être dues à des différences dans les étapes de production, d'isolement ou de purification de la ST aussi bien qu'à différents processus enzymatiques lorsque l'entérotoxine quitte la cellule.

Des études récentes (R.A. Kapitany et al., Infect. Immun. *26,* 173—177, 1979) soulèvent la question de l'hétérogénéité de la ST mais la découverte de différents types de ST basés — comme indiquée par R.A. Kapitany et al. (référence citée) — sur la composition en acides aminés, les propriétés de thermostabilité et l'activité biologique est sans aucune incidence sur la présente invention, laquelle est indistinctement applicable à chaque composition en acides aminés des différentes entérotoxines ST.

Différents milieux et méthodes d'incubation ont été utilisés par différents investigateurs lors de la culture des différentes souches de *E. coli* productrices de ST. Comme exemples de tels milieux, on peut citer le milieu complexe de F. G. Evans et al. (Infect. Immun. *8,* 731—35, 1973) et le milieu défini comme minimal de J. F. Alderete et al. (Infect. Immun. *15,* 781—88, 1977) ainsi que leurs modifications.

Dans les exemples suivants, l'entérotoxine ST a été préparée à partir d'une souche de *E. coli* K 12 qui a été déposée le 13 mars 1980 à l'American Type Culture Collection (Rockville, Maryland, Etats-Unis d'Amérique) sous le numéro ATCC 31621.

La souche de *E. coli* ATCC 31621 est une souche mutante dérivée de la souche bien connue *E. coli* K 12, isolée en 1922 des selles d'un convalescent qui avait contracté la diphtérie et utilisée universellement en recherche fondamentale en génétique bactérienne et en biologie moléculaire (B.J. Bachmann, Bacteriol. Rev. *36,* 4, 525, 1972). *E. coli* K 12 est une bactérie Gram négatif en forme de bâtonnet, d'environ deux $\mu$m de longueur et un $\mu$m de diamètre. Sa meilleure température de croissance est 37° C mais la souche croît et se multiplie également à des températures aussi basses que 20° C. *E. coli* K 12 est une bactérie mobile non capsulaire de sérotype O⁻ : K (?): sérotype H 48; elle manifeste une sensibilité très spécifique vis-à-vis du phage lambda.

La souche de *E. coli* ATCC 31621 présente les caractéristiques types de *E. coli* K 12 indiquées ci-dessus et, de plus, elle a des besoins auxotrophes en phénylalanine, tryptophane, histidine et proline et ne fermente pas le lactose, probablement à cause d'une mutation *lac* y (elle forme des colonies blanches sur le milieu lactose de Mc Conkey). La souche *E. coli* ATCC 31621 héberge un plasmide recombinant ayant un fragment ADN de 2, 1 × 10⁶ daltons obtenu par digestion par l'endonucléase de restriction d'un plasmide ADN provenant à l'origine d'une souche porcine pathogène de *E. coli* et comportant un gène codant pour la synthèse de l'entérotoxine thermostable. La souche est résistance au chloramphénicol (25 $\mu$g par ml).

Comme indiqué ci-dessus, l'invention n'est limitée ni à l'utilisation de la souche *E. coli* ATCC 31621 ni au procédé de fermentation décrit ci-après, ladite souche ainsi que les conditions de fermentation étant purement exemplatives pour la production de l'entérotoxine ST.

La présente invention concerne un procédé pour la préparation de dérivés de l'entérotoxine ST, lequel procédé comprend la réaction de l'entérotoxine ST avec un agent réticulant bifonctionnel pour protéines, et les dérivés de l'entérotoxine ST ainsi obtenus correspondent donc à une entérotoxine ST réticulée inter- ou intramoléculairement et présentant un poids moléculaire beaucoup plus élevé que l'entérotoxine elle-même.

Dans le procédé de la présente invention, on fait réagir l'entérotoxine ST avec un agent réticulant bifonctionnel atoxique pour protéines et agissant comme réactif homofonctionnel ou hétérofonctionnel pour donner une entérotoxine ST réticulée; comme exemples d'agents réticulants bifonctionnels pour protéines agissant comme réactifs homofonctionnels, on peut citer les dialdéhydes, les dicétones, les carbodiimides, les diisocyanates, les diisothiocyanates, les diazotures d'aryle, les diazotures d'acyle, les difluorures d'aryle (activé), les chlorures ou bromures d'arène disulfonyle (activé), les azotures, chlorures ou esters d'acide dicarboxylique (activé), les di-alpha halocétones, les esters d'acide polyméthylène (n=3—12) diimidique, les éthers bis-maléidométhyliques les gem-bis-diazoacétyl alcanes, les bis-diazoarènes et N,N'-arylène dimaléimides, et comme exemples de réactifs hétéro-fonctionnels, on peut citer ceux qui présentent deux groupes fonctionnels différents mais compatibles parmi ceux des agents réticulants ci-dessus, plus particulièrement, les isocyanato- et isothiocyanato-arènes, les fluoro- et azido-arènes (activés), les azido et fluoroarènes (activés), les azido-aryl alpha-haloalkyl cétones (activées), les esters alcoyliques inférieurs de l'acide chloro- ou bromo-acétimidique, les esters alcoyliques inférieurs de l'acide chloroformique et les épihalohydrines.

Les agents réticulants bifonctionnels pour protéines auxquels on se réfère dans la présente description sont bien connus des spécialistes. Par exemple, une revue des réticulations artificielles dans le domaine des protéines a été publiée par Rosa Uy et Finn Wold au chapitre 9 de "Advances in Experimental Medicine and Biology" intitulé "Protein Crosslinking (Biochemical and Molecular Aspects)" édité par Mendel Friedman, Plenum Press, New York.

Parmi ces différents agents réticulants, les plus généralement utilisés pour des raisons protiques sont les réactifs homofonctionnels tels que le glutaraldéhyde, le difluorodinitrobenzène, les composés diisocyanate et diisothiocyanate, les carbodiimides et les esters d'acide diimidique.

De tels agents réticulants ont déjà été utilisés pour réaliser l'agrégation de toxines afin d'obtenir des produits détoxifiés. Ainsi, le brevet français (certificat d'addition) No 2 270 891 revendique un procédé consistant à mettre en contact le produit toxique avec du glutaraldéhyde afin d'obtenir un produit détoxifié utilisable comme vaccin et le brevet allemand 2 137 630 revendique l'utilisation d'un diisocyanate dans le même but.

Néanmoins, le procédé n'avait, jusqu'ici, pas encore été appliqué à l'entérotoxine ST. En principe, la détoxification de l'entérotoxine ST ne présente d'ailleurs en elle-même aucun intérêt pratique pour la préparation de vaccins étant donné que la toxine de départ elle-même n'est pas antigénique.

Dans la présente invention, l'application de la méthode d'agrégation à l'aide d'agents réticulants bifonctionnels à l'entérotoxine ST est utilisée à une fin nouvelle et produit un effet nouveau et surprenant: en effet, alors que, jusqu'ici, l'utilisation d'agents réticulants servait à détoxifier un produit antigénique, dans la présente invention, elle permet d'obtenir un produit antigénique au départ d'un produit qui ne l'est pratiquement pas et le produit nouveau qui est ainsi obtenu constitue le premier vaccin spécifiquement efficace contre la diarrhée due à l'entérotoxine ST.

En conséquence, la présente invention concerne un procédé pour la préparation de dérivés de l'entérotoxine ST de *E. coli*, lequel procédé consiste à faire réagir l'entérotoxine ST avec un agent réticulant pour protéines.

L'entérotoxine ST de *E. coli* utilisée comme matière de départ dans le procédé de l'invention peut être obtenue à partir de n'importe quelle souche de *E. coli* productrice d'entérotoxine ST.

L'entérotoxine ST obtenue, présente dans le surnageant de culture, est ensuite généralement extraite et purifiée par élimination de certains contaminants, par exemple, par ultrafiltration, chromatographie par filtration sur gel, chromatographie d'échange ionique, extraction à l'éthanol ou électrophorèse sur gel de polyacrylamide. Une description de quelques-unes de ces techniques peut être obtenue dans un article publié par Y. Takeda et al. dans Infect. Immun. *25,* 978—985, 1979.

Etant donné que l'entérotoxine ST est assez bien thermorésistante, le procédé de l'invention peut s'effectuer dans une large gamme de températures, quoique, en pratique, on préfère opérer à une température inférieure à 37° C et plus spécialement à température ordinaire, c'est-à-dire à environ 20—25° C.

La réaction s'effectue en milieu aqueux et, de préférence, dans un milieu tamponné, à un pH dépendant de la nature de l'agent réticulant: par exemple, dans le cas des dialdéhydes ou des dicétones, le pH du milieu réactionnel n'est pas critique et une zone adéquate de pH est d'environ 4 à environ 8, un pH d'environ 5 étant préférable et de très basses valeurs de pH étant à éviter de manière à réduite l'auto-condensation possible de l'aldéhyde. Un pH de 5 s'avère également convenable pour les agents réticulants carbodiimide et di isocyanate mais pour l'épihalohydrine, un pH faiblement alcalin (p. ex. environ pH 8) est souhaitable.

Lorsqu'on emploie un dialdéhyde ou une dicétone la réticulation de l'entérotoxine ST présente en faible concentration dans le milieu, la réaction de réticulation peut être améliorée en précipitant la ST par la technique du relargage — par ex. par addition de sulfate de sodium au milieu — avant d'y ajouter l'agent réticulant.

La solubilité des dérivés d'entérotoxine ST obtenus est influencée par les différents degrés possibles de réticulation.

Les dérivés d'entérotoxine ST de l'invention peuvent être présentés sous la forme de compositions pharmaceutiques ou vétérinaires utilisables pour administration intramusculaire ou orale par addition d'un excipient acceptable pour une telle administration. Constituent des excipients convenables les solutions isotoniques et tamponnées et autres substances bien connues dans la technique.

Selon la solubilité du dérivé d'entérotoxine ST en milieu aqueux, la composition pharmaceutique ou vétérinaire administrée peut être une solution ou une suspension; néanmoins, on peut y ajouter un adjuvant tel que l'adjuvant complet de Freund (à usage vétérinaire), de l'hydroxyde d'aluminium ou tout autre adjuvant du même genre et, en pratique, la composition sera conservée sous forme lyophilisée et la solution (ou suspension) sera reconstituée extemporanément, p. ex. par addition d'adjuvant complet de Freund. Une dose unitaire de dérivé d'entérotoxine ST selon l'invention contient par exemple 0,1 mg de ST réticulée par kg de poids.

Les dérivés d'entérotoxine ST de l'invention peuvent être également utilisés à d'autres fins, p. ex. pour la préparation aisée d'antisérums anti-ST utiles pour les tests d'immunité.

## Exemple 1

On réhydrate un échantillon de la souche *E. coli* ATCC 31621 avec une solution saline stérile et on l'incube à 37° C pendant 18 heures dans des boîtes de Petri contenant chacune 20 ml de milieu solide "Selective LB agar" (un produit fabriqué et vendu par les Laboratoires DIFCO, Détroit 1, Michigan, E.U.A.) additionné de 25 $\mu$g de chloramphénicol par ml et préalablement chauffé pendant 45 minutes à 115° C.

On prépare ensuite un milieu de culture liquide (appelé PP3) en dissolvant 30 g de Proteose peptone N° 3 (un produit fabriqué et vendu par les Laboratoires DIFCO), 4 g d'extrait de levure et 5 g de dextrose dans un litre d'eau à 60° C. Après refroidissement, on y ajoute 5 g de NaCl, 5,05 g de $Na_2HPO_4$ et 1,2 g de $KH_2PO_4$. On filtre le milieu (de pH 6,9—7,0) sur filtre Seitz EKS et on le répartit en parties aliquotes de 30 ml dans des flacons de culture de 250 ml.

On ensemence ces flacons de culture avec les colonies obtenues sur boîtes de Pétri, en utilisant 4 colonies par 30 ml de milieu liquide PP3 et on les incube à 37° C pendant sept heures en les agitant sur des plaques oscillantes (20 à 30 oscillations par minute).

On procède à un second passage (pendant 16 heures) dans 800 ml de milieu PP3 contenu dans un flacon conique de 6 l en utilisant un inoculat à 5% (v/v) issu du premier passage.

On utilise la préculture obtenue comme inoculat (5% v/v) pour l'étape de production exécutée en fermenteur contenant 80 l de milieu Alderete modifié ($K_2HPO_4$ 8,71 g; NaCl 2,35 g; $NH_4Cl$ 1,0 g; tricine 1,8 g; $MgSO_4$. 7 aq. 50 mg; $MnCl_2$.4 aq. 4,95 mg et $FeCl_3$ 4,9 mg dans un litre d'eau) stérilisé à 121° C pendant 30 minutes et additionné ensuite de l-proline (1,42 g); d'acide aspartique (0,87 g); de l-alanine (0,39 g); de l-sérine (0,69 g); de l-phénylalanine (0,05 g); de l-histidine (0,05 g); de l-tryptophane (0,05 g) et de 5 g de Casamino acids (un produit fabriqué et vendu par les Laboratoires DIFCO, Détroit 1, Michigan, E.U.A.) pour un litre de solution finale, les acides aminés étant préparés séparément en solution 10 fois concentrée, ajustés à pH 7,5 et stérilisés à 121° C pendant 30 minutes avant d'être incorporés à la solution initiale.

On incube le milieu de production en condition aérobie en l'agitant à 36° C pendant 10 à 15 heures, c'est-à-dire jusqu'à ce que le pH atteigne 8,2—8,4 et la consommation en oxygène approche de zéro.

Au terme de l'étape de production, on porte le bouillon de culture à 60° C pendant 30 minutes, on le centrifuge à 6000 g et on filtre le surnageant sur filtre Seitz EKS1.

On concentre ensuite l'entérotoxine ST et on la purifie par un procédé en 3 étapes, comme suit:

Dans la première étape, on concentre une aliquote de 40 litres du milieu de fermentation filtré jusqu'à un volume de 5 litres, par filtration sur membrane(s) Pellicon PTAC ayant un "cut-off" de 1000 (Pellicon PTAC est la marque déposée pour une ultra-membrane formée d'esters mixtes de cellulose,

4

fabriquée et vendue par Millipore Corporation, Bedford, Massachusetts, E.U.A.) et on procédé ensuite à une dialyse sur la même membrane Pellicon PTAC jusqu'à ce que la conductivité ait diminué jusqu'à 500 microsiemens. On concentre ensuite jusqu'à un litre la fraction qui a été retenue et on la lyophilise. Le produit lyophilisé est dénommé ST—MP.

Dans la seconde étape, on dissout 3 grammes d'une partie aliquote du produit ST—MP dans 150 ml d'eau et on refroidit la solution à 0—4° C. On y ajoute de l'acétone froide (1,5 litre), goutte à goutte, sous agitation que l'on maintient pendant 30 minutes après addition complète de l'acétone. On centrifuge le milieu à 3000 g et on concentre le surnageant sous pression réduite jusqu'à un volume de 30 ml auquel on ajoute 180 ml d'un mélange méthanol/chloroforme 2:1 (v/v). Après vigoureuse agitation, on ajoute 42 ml d'eau et on sépare la couche supérieure; on l'évapore à sec sous pression réduite et on la lyophilise. Le produit lyophilisé est appelé ici ST—ACF—D.

Dans la troisième étape, on dissout 200 mg d'une partie aliquote de ST—ACF—D dans 2 ml d'eau et on l'introduit au sommet d'une colonne réfrigérée (2,5 × 30 cm) contenant 50 g de Servachrom-8 (Servachrom-8 est la marque déposée d'un ester méthylique de l'acide polyacrylique pour analyse chromatographique vendu par SERVA G.m.b.H. et Co. Heidelberg, République Fédérale d'Allemagne) équilibrée avec de l'eau. On procède à l'élution avec de l'eau. Après élution de la première fraction, on poursuit l'élution avec un mélange de méthanol/eau 6:4 (v/v) afin d'éluer la fraction contenant la ST. On isole la ST par évaporation du solvant et le résidu ainsi obtenu est appelé ST—PAE.

La ST—PAE présente un facteur de purification de 1000—4000 par rapport à l'activité du lot initial, l'activité spécifique de produit ST—PAE variant entre 5,5 et 15 nanogrammes de lot à lot.

On détermine l'activité biologique des fractions au moyen du test sur souriceaux selon la méthode décrite par A.G. Dean (J. Infect. Dis. *125,* 407—411, 1972). A cette fin, on administre par voie intragastrique aux souriceaux âgés de 48—72 heures, une solution contenant 0,025 ml de toxine et, deux heures plus tard, on mesure l'accumulation du liquide dans l'intestin. Le rapport

$$\frac{\text{poids de l'intestin}}{\text{poids corporel}}$$

est une mesure de l'activité biologique de la toxine. Les valeurs supérieures ou égales à 0,09 sont considérées comme positives. L'activité spécifique de la fraction ST est ensuite exprimée comme étant la quantité de ST (en nanogrammes de produit lyophilisé) qui donne une valeur

$$\frac{\text{poids de l'intestin}}{\text{poids corporel}}$$

de 0.100 dans le test sur souriceaux.

## Exemple 2

On dissout 10 mg de ST—PAE dans 0,25 ml de tampon acétate (pH 5,1) et on la précipite par addition de 0,25 ml d'une solution de sulfate de sodium à 36% (p/v) dans le même tampon. On ajoute une solution aqueuse de glutaraldéhyde (20 microlitres d'une solution à 25% v/v) et on laisse réagir le mélange pendant deux heures à température ordinaire. Après ce temps de réaction, on sépare par centrifugation et lavage à l'eau le réactif en excès et on lyophilise le précipité.

Le produit lyophilisé consiste en ST réticulée immunogène de poids moléculaire accru par rapport à la ST utilisée comme produit de départ, comme l'indique la chromatographie sur gel de Sephadex G—100 (Sephadex est la marque déposée d'une préparation de dextran pour analyse chromatographique fabriquée et vendue par Pharmacia, Uppsala, Suède).

Pour démontrer l'augmentation du poids moléculaire, on solubilise partiellement le produit lyophilisé en ajoutant un ml d'eau et en agitant le mélange pendant deux heures à température ordinaire. La fraction solubilisée représente de 30 à 50% de la fraction insoluble et indique un pic principal à 10.000 alors que, dans les mêmes conditions, le produit de départ ST—PAE indique un pic principal à 4000 (pic en arrière de Vt sur Sephadex G—100, Vt étant le volume de la colonne).

## Exemple 3

On dissout 20 mg de ST—PAE dans un ml d'eau et on y ajoute une solution de 200 mg de chlorhydrate de 1-éthyl-3(3-diméthylaminopropyl)-carbodiimide. On laisse reposer le mélange à 25° C pendant 12 heures pendant lesquelles la réaction de réticulation se produit. On dialyse le produit de réaction soluble sur une membrane Amicon PM—10 (Amicon est la marque déposée d'une ultramembrane fabriquée et vendue par Amicon Corporation, Lexington, Massachusetts, Etats-Unis d'Amérique) afin d'éliminer la ST qui n'a pas réagi et on lyophilise la fraction retenue (13 mg). Le poids moléculaire de cette ST réticulée est d'environ 10.000 (par rapport à 2000 pour le produit de départ) lorsqu'il est déterminé par chromatographie sur gel de Sephadex G—100, comme indiqué à l'exemple 2. Le spectre

**0 037 522**

d'absorption de cette ST réticulée est également différent du spectre d'absorption de la fraction ST—PAE employée comme produit de départ.

### Exemple 4

On dissout 5 mg de ST—PAE dans 0,5 ml de tampon borate 0,1 mol/1 ($Na_2B_4O_7$/NaOH) à pH 8,0, et on y ajoute 100 microlitres de difluorodinitrobenzène dans de l'éthanol. On laisse évoluer la réaction de réticulation, sous agitation, pendant 4 heures à température ordinaire. Après ce temps de réaction, la ST qui n'a pas réagi est éliminée par dialyse à travers un tube à dialyse Spectrapor ayant un "cut off" de 14000 (Spectrapor est la marque déposée d'un tube à dialyse fabriqué et vendu par Spectrum Medical Industries Inc., New York, Etats-Unis d'Amérique).

Par chromatographie sur gel de Sephadex G—100 (comme indiqué à l'exemple 2), le poids moléculaire de cette ST réticulée soluble est estimé à 50.000 (par rapport à 2000 pour la ST—PAE de départ). Le spectre d'absorption de la ST réticulée obtenue indique également un pic supplémentaire à 350 nanomètres par rapport au spectre d'absorption de la ST—PAE de départ.

### Exemple 5

Dans 0,5 l de sérum physiologique et 0,5 litre d'adjuvant complet de Freund, on met en suspension 100 mg de dérivé d'entérotoxine ST lyophilisé comme obtenu à la fin de l'exemple 2 et on répartit la composition dans des flacons en verre contenant chacun une dose effective d'entérotoxine ST réticulée pour administration vétérinaire. Les flacons sont alors fermés hermétiquement et conservés à 4° C.

### Exemple 6

Dans un litre de sérum physiologique et 10 g d'Alhydrogel (un produit fabriqué et vendu par Supergod Export Company, Copenhague, Danemark), on met en suspension 100 mg de dérivé d'entérotoxine ST lyophilisé, comme obtenu à la fin de l'exemple 2 et on répartit la composition dans des flacons en verre contenant chacun une dose effective d'entérotoxine ST réticulée pour administration humaine. Les flacons sont alors fermés hermétiquement et conservés à 4° C.

### Exemple 7

On administre, par voie sous-cutanée, à trois lapins (A, B et C) une composition comme préparée à l'exemple 5, à raison d'une dose unitaire de 0,1 mg par animal et on répète la même administration sous-cutanée 21 jours plus tard. On prélève des échantillons sanguins (c'est-à-dire d'antisérum) le vingt-huitième jour après la première administration.

On teste les sérums des lapins immunisés pour leur capacité à neutraliser (*in vitro,* 2 heures à 37° C) une quantité fixe de ST, comme déterminé par le test sur souriceaux (A.G. Dean, *Loc. cit.*); comme témoin, à la place d'antisérum, on emploie un tampon phosphate au sérum physiologique ou du sérum normal.

Les résultats du test de séroneutralisation sont donnés dans le Tableau I suivant dans lequel
— PBS signifie une solution tampon comprenant 8 g de NaCl; 0,2 g de KCl; 1,15 g de $Na_2HPO_4$; 0,2 g de $KH_2PO_4$ dans de l'eau distillée jusqu'à 800 ml mélangée à une solution de 100 mg de $MgCl_2$.6 aq. dans 100 ml d'eau distillée et ensuite à une solution de 0,1 g de $CaCl_2$ dans 100 ml d'eau.
— les valeurs de pi/pc inférieures à 0,09 sont une indication de la neutralisation de la ST.
— chaque valeur pi/pc (poids de l'intestin/poids corporel) est une valeur moyenne correspondant à 3 déterminations, chacune d'elles représentant un rapport pi/pc d'un groupe de 4 souriceaux.
— les volumes de ST se rapportent à une solution de ST contenant 1,4 $\mu$g de toxine de pureté ST—ACF—D par ml.

6

**0 037 522**

TABLEAU I

| Schéma | Dilution de l'antisérum | pi/pe |
|---|---|---|
| Témoin<br>0,5 ml ST + 0,5 ml PBS | — | 0,102 |
| Lapin A<br>0,5 ml ST + 0,5 ml antisérum | 1/16 | 0,064 |
| | 1/32 | 0,087 |
| | 1/128 | 0,097 |
| Lapin B<br>0,5 ml ST + 0,5 ml antisérum | 1/4 | 0,059 |
| | 1/8 | 0,100 |
| Lapin C<br>0,5 ml ST + 0,5 ml antisérum | 1/8 | 0,059 |
| | 1/16 | 0,098 |

Les résultats démontrent l'immunogénicité de la ST réticulée.

### Exemple 8

On administre par voie sous-cutanée, à 3 lapins (K,L et M) une composition comme préparée à l'exemple 5 mais avec la toxine réticulée obtenue à l'exemple 3 (fraction soluble ayant un poids moléculaire supérieur à 10.000), à raison d'une dose unitaire de 0,1 mg par animal et on répète la même administration sous-cutanée 21 jours plus tard. On prélève des échantillons sanguins (c'est-à-dire d'antisérum) le 28ème jour après la première administration et on effectue les tests de séroneutralisation comme dans l'exemple 7 mais en utilisant du sérum normal au lieu de PBS. Les résultats sont indiqués au Tableau II et montrent l'immunogénicité de la ST réticulée dans un animal sur trois.

TABLEAU II

| Schéma | Dilution de l'antisérum | pi/pe |
|---|---|---|
| Témoin<br>0,5 ml ST + 0,5 ml sérum | — | 0,129 |
| Lapin L<br>0,5 ml ST + 0,5 ml antisérum | 1/1 | 0,060 |
| Lapin K<br>0,5 ml ST + 0,5 ml antisérum | 1/1 | 0,100 |
| Lapin M<br>0,5 ml ST + 0,5 ml antisérum | 1/1 | 0,111 |

### Exemple 9

On administre par voie sous cutanée, à 3 lapins (H, I et J), une composition comme préparée à l'exemple 5 mais avec la toxine réticulée comme obtenue à l'exemple 4 (fraction soluble ayant un poids moléculaire supérieur à 14.000), à raison d'une dose unitaire de 0,5 mg par animal et on répète la même administration sous-cutanée 21 jours plus tard. On prélève des échantillons sanguins (c'est-à-dire d'antisérum) le 28ème jour après la première administration et on effectue les tests de séro neutralisation comme dans l'exemple 7. Les résultats sont donnés dans le tableau III suivant, démontrant l'immunogénicité de la ST réticulée.

**0 037 522**

TABLEAU III

| Schéma | Dilution de l'antisérum | pi/pe |
|---|---|---|
| Témoin 0,5 ml ST + 0,5 ml PBS | — | 0,114 |
| Lapin H 0,5 ml ST + 0,5 ml antisérum | 1/1 | 0,078 |
| Lapin I 0,5 ml ST + 0,5 ml antisérum | 1/1 | 0,073 |
| Lapin J 0,5 ml ST + 0,5 ml antisérum | 1/1 | 0,061 |

Exemple 10

On administre, par voie sous-cutanée, à trois porcelets (de poids corporel 20 kg) une composition comme préparée à l'exemple 5, à raison d'une dose unitaire de 0,5 mg (jour 0), 0,5 mg (21ème jour) et 5 mg (46ème jour) pour les porcelets A et B et 0,1 mg (jour 0), 1 mg (21ème jour) et 5 mg (46ème jour) pour le porcelet C. On prélève des échantillons sanguins (c'est-à-dire d'antisérum) le 61ème jour et on effectue les tests de séroneutralisation comme décrit dans l'example 7.

Les résultats sont indiqués dans le tableau IV suivant et démontrent l'immunogénicité de la ST réticulée.

TABLEAU IV

| Schéma d'immunisation | Dilution de l'antisérum | pi/pe |
|---|---|---|
| Témoin 0,5 ml ST + 0,5 ml PBS | — | 0,111 |
| Porcelet A | 1/32 | 0,054 |
| 0,5 ml ST + 0,5 ml antisérum | 1/64 | 0,062 |
| | 1/28 | 0,920 |
| Porcelet B | 1,8 | 0,065 |
| 0,5 ml ST + 0,5 ml antisérum | 1/16 | 0,076 |
| | 1/32 | 0,108 |
| Porcelet C | 1/16 | 0,055 |
| 0,5 ml ST + 0,5 ml antisérum | 1/32 | 0,059 |
| | 1/64 | 0,095 |

**Revendications pour les Etats contractants: BE CH DE FR GB IT LI LU NL SE**

1. Procédé de préparation de dérivés d'une toxine par réaction de ladite toxine avec un agent réticulant bifonctionnel atoxique pour protéines et isolement des dérivés de toxine ainsi obtenus, caractérisé en ce que la toxine est l'entérotoxine ST de *E. coli.*

2. Procédé suivant la revendication 1, dans lequel l'agent réticulant bifonctionnel est un dialdéhyde, une dicétone, un carbodiimide, un diisocyanate, une épihalohydrine ou un difluorure.

3. Dérivés de l'entérotoxine ST de *E. coli* caractérisés en ce qu'ils sont obtenus par le procédé de la revendication 1.

4. Dérivés de l'entérotoxine ST de *E. coli* caractérisés en ce qu'ils sont obtenus par réticulation d'une entérotoxine de *E. coli* avec un dialdéhyde, une dicétone, un carbodiimide, un diisocyanate, une épihalohydrine ou un difluorure.

5. Composition destinée à immuniser un organisme humain ou animal contre une diarrhée due à

8

# O 037 522

une infection par entérotoxine de *E. coli,* caractérisée en ce qu'elle comprend un dérivé d'entérotoxine ST de *E. coli,* obtenu par le procédé de la revendication 1 et un excipient pharmaceutique ou vétérinaire pour l'administration intramusculaire, sous-cutanée ou orale, avec un adjuvant.

**Revendications pour l'Etat contractant: AT**

1. Procédé de préparation de dérivés d'une toxine par réaction de ladite toxine avec un agent réticulant bifonctionnel atoxique pour protéines et isolement des dérivés de toxine ainsi obtenus, caractérisé en ce que la toxine est l'entérotoxine ST de *E. coli.*

2. Procédé suivant la revendication 1, dans lequel l'agent réticulant bifonctionnel est un dialdéhyde, une dicétone, un carbodiimide, un diisocyante, une épihalohydrine ou un difluorure.

3. Procédé suivant la revendication 1 tel que décrit dans l'un ou l'autre des exemples 1, 2, 3 ou 4.

**Patentansprüche für die Vertragsstaaten: BE CH DE FR GB IT LI LU NL SE**

1. Verfahren zur Herstellung von Derivaten eines Toxins durch Umsetzung des Toxins mit einem bifunktionellen, nichttoxischen Vernetzungsmittel für Proteine und Gewinnung der so erhaltenen Toxinderivate, dadurch gekennzeichnet, daß das Toxin das Enterotoxin ST von *E. coli* ist.

2. Verfahren nach Anspruch 1, wobei das bifunktionelle Vernetzungsmittel ein Dialdehyd, ein Diketon, ein Carbodiimid, ein Diisocyanat, ein Epihalogenhydrin oder ein Difluorid ist.

3. Derivate des Enterotoxins ST von *E. coli,* dadurch gekennzeichnet, daß sie nach dem Verfahren von Anspruch 1 erhalten worden sind.

4. Derivate des Enterotoxins ST von *E. coli,* dadurch gekennzeichnet, daß sie durch Vernetzung eines Enterotoxins von *E. coli* mit einem Dialdehyd, einem Diketon, einem Carbodiimid, einem Diisocyanat, einem Epihalogenhydrin oder einem Difluorid erhalten worden sind.

5. Mittel zur Immunisierung eines menschlichen oder tierischen Organismus gegen eine durch eine Infektion mit Enterotoxin von *E. coli* verursachte Diarrhoe, dadurch gekennzeichnet, daß es ein Derivat des Enterotoxins ST von *E. coli,* erhalten nach dem Verfahren von Anspruch 1, und einen pharmazeutischen oder veterinärmedizinischen Träger für die intramuskuläre, subcutane oder orale Verabreichung, zusammen mit einem Hilfsmittel umfaßt.

**Patentansprüche für den Vertragsstaat: AT**

1. Verfahren zur Herstellung von Derivaten eines Toxins durch Umsetzung des Toxins mit einem bifunktionellen, nichttoxischen Vernetzungsmittel für Proteine und Gewinnung der so erhaltenen Toxinderivate, dadurch gekennzeichnet, daß das Toxin das Enterotoxin ST von *E. coli* ist.

2. Verfahren nach Anspruch 1, wobei das bifunktionelle Vernetzungsmittel ein Dialdehyd, ein Diketon, ein Carbodiimid, ein Diisocyanat, ein Epihalogenhydrin oder ein Difluorid ist.

3. Verfahren nach Anspruch 1, wie in irgendeinem der Beispieeele 1, 2, 3 oder 4 beschrieben.

**Claims for the Contracting States: BE CH DE FR GB IT LI LU NL SE**

1. Process for the preparation of toxin derivatives by reaction of said toxin with a non toxic bifunctional crosslinking agent for proteins and isolation of the toxin derivatives so-obtained, characterised in that the toxin is the *E. coli* ST enterotoxin.

2. Process according to claim 1 wherein the non toxic bifunctional crosslinking agent is a dialdehyde, a diketone, a carbodiimide, a diisocyanate, an epihalohydrin or a difluoride.

3. *E. coli* St enterotoxin derivatives, characterised in that they are obtained by the process of claim 1.

4. *E. coli* ST enterotoxin derivatives characterised in that they are obtained by crosslinking of an *E. coli* enterotoxin with a dialdehyde, a diketone, a carbodiimide, a diisocyanate, an epihalohydrin or a difluoride.

5. Composition for immunizing a human or animal organism against diarrhea provoked by an infection with *E. coli* enterotoxin, characterised in that it comprises an *E. coli* ST enterotoxin derivative obtained by the process of claim 1 and pharmaceutical or veterinary carrier for intramuscular, subcutaneous or oral administration, with an adjuvant.

**Claims for the Contracting State: AT**

1. Process for the preparation of toxin derivatives by reaction of said toxin with a non toxic bifunctional crosslinking agent for proteins and isolation of the toxin derivatives so-obtained, characterised in that the toxin is the *E. coli* ST enterotoxin.

2. Process according to claim 1 wherein the non toxic bifunctional crosslinking agent is a dialdehyde, a diketone, a carbodiimide, a diisocyanate, an epihalohydrin or a difluoride.

3. Process according to claim 1 as described in any of examples 1, 2, 3 or 4.